# EUROPÄISCHE PATENTANMELDUNG

(11) **EP 1 743 609 A1**
(43) Veröffentlichungstag der Anmeldung: **17.01.2007**
(21) Anmeldenummer: 05015082.0
(22) Anmeldetag: 12.07.2005
(51) Int. Cl.: A61F 9/009, A61F 9/013

(54) **Einrichtung für die Augenchirurgie**

(71) Anmelder: WaveLight Laser Technologie AG, 91058 Erlangen (DE)
(72) Erfinder: Aziret, Sinan, 90441 Nürnberg (DE); Donitzky, Christof, 90542 Eckental (DE)
(74) Vertreter: von Hellfeld, Axel

(57) **Zusammenfassung**

Eine Einrichtung für die refraktive Augenchirurgie umfasst eine Vakuumpumpanordnung (10, 12), ein von der Vakuumpumpanordnung ausgehendes Evakuierungswegsystem (16) zum Anschluss an eine auf das Auge aufzusetzende Komponente (18) sowie Drucksteuermittel (24) zur Steuerung des Drucks in dem Evakuierungswegsystem. Stromabwärts der Vakuumpumpanordnung (10, 12) ist in das Evakuierungswegsystem (16) eine steuerbare Fremdlufteinleitungsstelle (22) eingefügt, wobei die Drucksteuermittel (24) mit der Fremdlufteinleitungsstelle gekoppelt sind und dazu eingerichtet sind, zur Steuerung des Drucks in dem Evakuierungswegsystem (16) den Fremdlufteinleitungsquerschnitt der Fremdlufteinleitungsstelle (22) zu beeinflussen.

## Beschreibung

Die Erfindung betrifft eine Einrichtung für die Augenchirurgie, mit einer Vakuumpumpanordnung, einem von der Vakuumpumpanordnung ausgehenden Evakuierungswegsystem zum Anschluss an eine auf das Auge aufzusetzende Komponente und Drucksteuermittein zur Steuerung des Drucks in dem Evakuierungswegsystem.

Eine derartige. Einrichtung findet insbesondere bei der refraktiven Augenchirurgie Anwendung, bei der zur Korrektur von Sehfehlern die Hornhaut neugeformt wird. Bei modernen Techniken der refraktiven Augenchirurgie wird ein dünner Flap der Hornhaut von darunterliegenden Hornhautbereichen gelöst, allerdings nicht vollständig, sondern so, dass er noch an diesen darunterliegenden Hornhautbereichen hängt. Nach Wegklappen des Flaps werden die so freigelegten Hornhautbereiche durch Ablation mittels eines Lasers, gemeinhin eines Excimer-Lasers, im Sinne einer Behebung oder zumindest Reduzierung des Sehfehlers neugeformt und anschließend der Flap wieder zurückgeklappt. Zur Loslösung des Flaps von der Hornhaut sind verschiedene Techniken bekannt. Es kann ein Mikrokeratom eingesetzt werden, das über die Hornhaut bewegt wird und dabei den Flap herausschneidet. Alternativ kann zunächst mittels eines Trepans, das ist ein Schneidrohr, das Epithel entlang eines Kreisbogens eingeschnitten werden und anschließend mittels einer Alkohollösung das Epithel losgelöst werden. Eine ohne jede mechanische Schneideinwirkung auskommende weitere Methode verwendet einen sogenannten Femtosekundenlaser. Dieser strahlt ultrakurze Laserlichtpulse aus, die in definiert einstellbarer Tiefe der Hornhaut eine Materialtrennung bewirken und so die Loslösung des Flaps gestatten.

Bei Verfahren mit Einsatz einer mechanischen Schneideinheit wird üblicherweise eine Saugringeinheit am Auge angebracht, die zur Aufnahme und Führung der Schneideinheit dient. Die Saugringeinheit weist an ihrer dem Auge zugewandten Seite eine Ringnut auf, die durch Aufsetzen der Saugringeinheit auf den Augapfel geschlossen wird. Durch Anschluss der Saugringeinheit an eine Vakuumpumpe kann die Ringnut evakuiert werden. Hierdurch saugt sich die Saugringeinheit am Augapfel fest und sorgt für dessen Fixierung.

Die Saugringeinheit liegt allerdings normalerweise nicht perfekt dicht am Augapfel an. Stattdessen können zwischen Saugringeinheit und Augapfel geringe Mengen Luft in den Evakuierungstrakt eintreten, so dass zur Aufrechterhaltung des Vakuums die Vakuumpumpe auch nach erstmaligem Ansaugen der Saugringeinheit weiter betätigt werden muss. Bei einer bekannte Variante wird dabei das Ist-Vakuum gemessen und mit einem gewünschten Soll-Vakuum verglichen. Ist das Ist-Vakuum um mindestens einen vorbestimmten Prozentsatz schwächer als das Soll-Vakuum, wird die Vakuumpumpe eingeschaltet (ohne Regelung der Pumpleistung). Ansonsten bleibt die Vakuumpumpe ausgeschaltet. Bei einer anderen bekannten Variante wird die Pumpleistung der Vakuumpumpe im Sinne einer ständigen Angleichung des Ist-Vakuums an das gewünschte Soll-Vakuum geregelt.

Beide vorstehenden Varianten der Vakuumsteuerung haben sich als nachteilig erwiesen. Die Variante mit Ein- und Ausschalten der Vakuumpumpe führt zu einem pulsierenden Vakuum aufgrund des wiederkehrenden Einschaltens der Pumpe. Die entstehenden Pulsationen können vom Patienten als ausgesprochen unangenehm empfunden werden. Auch können sie der Präzision des Hornhautschnitts abträglich sein. Da die Schneideinheit, sei es ein Mikrokeratom oder ein Trepanrohr, an der Saugringeinheit gehalten und geführt wird, können sich Pulsationen des Vakuums über die Saugringeinheit auf die Schneideinheit übertragen und dementsprechend die Schnittqualität herabsetzen. Beispielsweise können pulsationsbedingte Schnittfehler zu Abweichungen von der gewünschten Dicke des abzulösenden Epithelflaps oder zu Abweichungen von der gewünschten Größe des Flaps führen.

Für beide Varianten gilt zudem, dass das Ansprechverhalten des Motors der Vakuumpumpe in der Regel nicht hinreichend dynamisch ist, um bei plötzlichem Vakuumabfall während der Operation rasch genug reagieren zu können. Es ist ohne weiteres möglich, dass die Saugringeinheit während der Operation Gefahr läuft, sich vom Auge abzulösen, etwa weil der Operateur sie versehentlich unsachgemäß handhabt oder mechanische Kippmomente auf sie einwirken. Auch kann in dem die Vakuumpumpe mit der Saugringeinheit verbindenden Schlauchsystem ein plötzliches Leck entstehen. In solchen Fällen ist es von enormer Wichtigkeit, dass rasch genug reagiert wird, um nicht durch mangelnde Präzision des Schnitts den Operationserfolg zu gefährden und womöglich das Auge nachhaltig zu beschädigen.

Aufgabe der Erfindung ist es daher, einen Weg aufzuzeigen, wie bei einer gattungsgemäßen Einrichtung ein Vakuum mit der bei der refraktiven Augenchirurgie verlangten hohen Qualität bereitgestellt werden kann.

Zur Lösung dieser Aufgabe sieht die Erfindung vor, dass eine steuerbare Fremdlufteinleitungsstelle stromabwärts der Vakuumpumpanordnung in das Evakuierungswegsystem eingefügt ist, wobei die Drucksteuermittel mit der Fremdlufteinleitungsstelle gekoppelt sind und dazu eingerichtet sind, zur Steuerung des Drucks in dem Evakuierungswegsystem den Fremdlufteinleitungsquerschnitt der Fremdlufteinleitungsstelle zu beeinflussen. Die erfindungsgemäße Lösung schlägt eine Abkehr von der bisherigen Übung vor, den Druck, d.h. das Vakuum, in dem Evakuierungswegsystem über die Vakuumpumpanordnung zu steuern. Stattdessen ermöglicht sie eine Drucksteuerung über eine speziell vorgesehene steuerbare Fremdlufteinleitungsstelle, deren Fremdlufteinleitungsquerschnitt die Güte des Vakuums in dem Evakuierungswegsystem beeinflusst. Die Fremdlufteinleitungsstelle stellt sozusagen ein gewolltes Leck im Evakuierungswegsystem dar. Ihr Querschnitt kann einfach mittels eines Schiebers bzw. Ventils variiert werden, zu dessen Verstellung wesentlich kleinere Massen bewegt werden müssen als bei Veränderung der Pumpleistung der Vakuumpumpanordnung. Deshalb kann der Fremdlufteinleitungsquerschnitt der Fremdlufteinleitungsstelle hochdynamisch an eine unerwünschte Veränderung des Ist-Zustands des in dem Evakuierungswegsystem herrschenden Vakuums angepasst werden, um die für den Operationserfolg benötigten gleichbleibenden Vakuumverhältnisse zu gewährleisten. Das Antwortverhalten des Gesamtsystems auf Schwankungen des Ist-Vakuums, aber auch auf ein verändertes Soll-Vakuum, kann so beträchtlich beschleunigt werden. Zweckmäßigerweise ist der Fremdlufteinleitungsquerschnitt der Fremdlufteinleitungsstelle stufenlos variierbar, um für eine äußerst feine Einstellbarkeit des Vakuums in dem Evakuierungswegsystem zu sorgen.

Im Rahmen eines Verfahrens zum Betreiben einer erfindungsgemäßen Einrichtung kann dann vorteilhaft mindestens eine Vakuumpumpe der Vakuumpumpanordnung kontinuierlich mit einer vorbestimmten Pumpleistung, insbesondere einer maximalen Pumpleistung, betrieben werden, wobei der Druck in dem Evakuierungswegsystem durch Beeinflussung des Fremdlufteinleitungsquerschnitts der Fremdlufteinleitungsstelle auf einen gewünschten Solldruckwert eingeregelt wird. Störende Pulsationen in dem Vakuum infolge wiederholten Ein- und Ausschaltens der Vakuumpumpe können so wirksam vermieden werden.

Bei einer vorteilhaften Weiterbildung der erfindungsgemäßen Einrichtung umfasst die Vakuumpumpanordnung zwei parallel geschaltete Vakuumpumpen. Durch einen stromabwärts der Vakuumpumpanordnung, jedoch stromaufwärts der Fremdlufteinleitungsstelle in das Evakuierungswegsystem eingefügten Umschalter kann dabei jede der Vakuumpumpen wahlweise zuschaltbar sein. Die Bereitstellung mehrerer Vakuumpumpen ermöglicht es, von einer Vakuumpumpe auf eine andere umzuschalten, sollte während einer Operation eine Pumpe ausfallen. Es muss dann nicht notwendig die Operation abgebrochen oder zumindest unterbrochen werden.

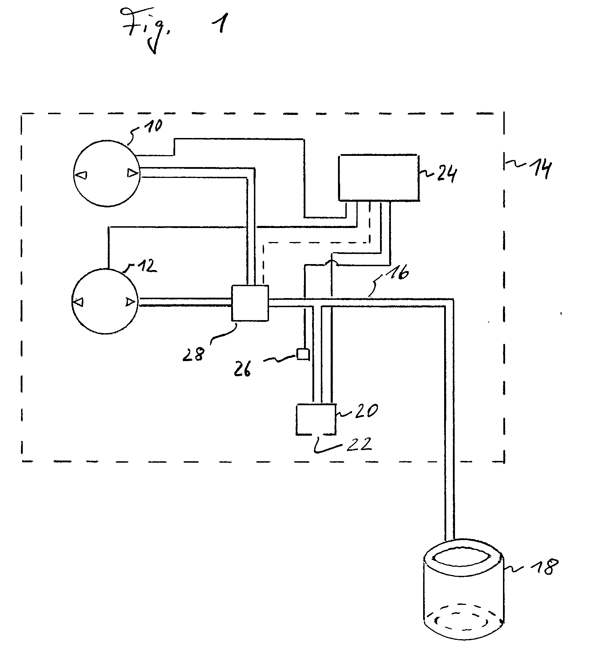

Ein Ausführungsbeispiel der Erfindung ist schematisch in der beigefügten Zeichnung dargestellt, deren einzige Fig. 1 eine Gesamtübersicht von Komponenten zeigt, die bei der Präparation eines Epithel (allgemein: Flap) zur anschließenden Neuformung der Augenhornhaut mittels Laserstrahlung, insbesondere unter Verwendung eines Excimer-Lasers, zum Einsatz kommen können. Diese Komponenten umfassen eine Pumpanordnung mit zwei parallel geschalteten Pumpen 10, 12, von denen eine als Hauptpumpe und die andere als bedarfsweise zuschaltbare Hilfspumpe dienen kann. Die Pumpen 10, 12 sind in einer schematisch durch Strichlinien angedeuteten, vorzugsweise als Tischgerät ausgebildeten Basiseinheit 14 untergebracht, in der verschiedene weitere Komponenten wie etwa eine Betriebsspannungsversorgung untergebracht sein können. Die Pumpen 10, 12 sind an ein allgemein mit 16 bezeichnetes, teilweise innerhalb der Basiseinheit 14 verlaufendes Evakuierungswegsystem angeschlossen, dessen außerhalb der Basiseinheit 14 verlaufende Teile beispielsweise von einer oder mehreren Schlauchleitungen gebildet sein können, die an einen oder mehrere geeignete Schlauchleitungsanschlüsse an der Außenseite der Basiseinheit 14 anschließbar sein können.

Eine an sich bekannte und deswegen nur grob schematisch angedeutete Saugringeinheit 18 ist an das Evakuierungswegsystem 16 anschließbar, um sie am Augapfel zu dessen Fixierung festzusaugen. An der Saugringeinheit 18 kann eine nicht näher dargestellte Schneideinheit linear- oder drehbeweglich geführt sein, mittels der zur Präparation des wegzuklappenden Epithelflaps/Flaps ein Schnitt in die Hornhaut eingebracht werden kann.

Es ist wichtig, dass während des Schnitts konstante Vakuumbedingungen in dem zu der Saugringeinheit 18 führenden Teil des Evakuierungswegsystems 16 herrschen, damit die Saugringeinheit 18 sicher am Augapfel haftet und diesen sicher fixieren kann. In das Evakuierungswegsystem 16 ist hierzu ein steuerbares Bypassventil 20 eingefügt, durch das Fremdluft von der Außenumgebung in das Evakuierungswegsystem 16 gelangen kann. Vorzugsweise ist das Bypassventil 20 ein Proportional-Wegeventil, das stufenlos zwischen einer vollständig geschlossenen Stellung, in der eine von dem Bypassventil 20 gebildete Bypassöffnung 22 geschlossen ist und dementsprechend keine Fremdluft durch die Bypassöffnung 22 in das Evakuierungswegsystem 16 eintreten kann, und einer vollständig geöffneten Stellung verstellbar ist, in der der Öffnungsquerschnitt der Bypassöffnung 22 maximal ist. Die Bypassöffnung 22 bildet eine Fremdlufteinleitungsstelle im Sinne der Erfindung.

Das Bypassventil 20 ist elektrisch ansteuerbar. Zu seiner Steuerung dienen geeignete elektrische oder/und elektronische Steuermittel 24, die in der Figur schematisch als ein Block dargestellt sind. Zweckmäßigerweise sind die Steuermittel 24 zusammen mit dem Bypassventil 20 in der Basiseinheit 14 untergebracht. Sie sind Teil eines Regelkreises für das Bypassventil 20, der den herrschenden Ist-Druck in dem zu der Saugringeinheit 18 führenden Teil des Evakuierungswegsystems 16 auf einen vorgegebenen Sollwert einregelt. Zur Erfassung des Ist-Drucks ist ein Drucksensor 26 vorgesehen, dessen Druckmesswert von den Steuermitteln 24 mit dem Drucksollwert verglichen wird. Die resultierende Regelabweichung wird von den Steuermitteln 24 in eine entsprechende Stellgröße für das Bypassventil 20 umgesetzt. Der Drucksollwert kann bedienerseitig vorgebbar sein oder/und in einem den Steuermitteln 24 zugeordneten elektronischen Speicher gespeichert sein.

Als Beispiel kann das Bypassventil 20 von einem unter der Handelsbezeichnung "SENTRONIC" vertriebenen 3-Wege-Proportionalventil der Fa. Asco Joucomatic gebildet sein. Dieses Proportionalventil enthält bereits einen Drucksensor, einen Soll/Ist-Vergleicher für den Druck sowie einen Umsetzer zur Umsetzung der Regelabweichung zwischen Soll- und Ist-Druck in einen Stellstrom für ein Elektromagnet-Stellglied des Proportionalventils als baulich integrierte Bestandteile. Die Steuermittel 24 sind bei Verwendung eines derartigen Proportionalventils somit zumindest teilweise mit dem Ventil in einer Baueinheit zusammengefasst. Es versteht sich freilich, dass das Bypassventil 20 alternativ von den übrigen Teilen des Regelkreises baulich getrennt sein kann.

Ein den Pumpen 10, 12 nachgeschalteter Umschalter 28 gestattet einen Wechsel zwischen den beiden Pumpen, so dass bei Ausfall der Hauptpumpe, etwa der Pumpe 10, jederzeit auf die Hilfspumpe, etwa die Pumpe 12, umgeschaltet werden kann. Es versteht sich, dass der Umschalter 28 so abgewandelt werden kann, dass er alternativ oder zusätzlich zu einem bloßen Wechsel zwischen den Pumpen 10, 12 ein Zuschalten der Hilfspumpe zu der Hauptpumpe ermöglichen kann, um so die Pumpleistung beider Pumpen nutzen zu können, sollte eine besonders hohe Gesamtpumpleistung benötigt werden. Der Umschalter 28 kann vom Bediener betätigbar sein, wozu an der Basiseinheit 14 ein entsprechendes Bedienelement vorgesehen sein kann. Alternativ oder zusätzlich kann der Umschalter 28 von den Steuermitteln 24 automatisch betätigbar sein, beispielsweise wenn die Steuermittel 24 mit Hilfe einer geeigneten Sensorik einen Ausfall der Hauptpumpe erkennen. Soll auf einen Umschalter verzichtet werden, können die beiden Pumpen direkt miteinander verbunden sein und elektrisch einzeln zu- und abschaltbar sein. Es werden die Pumpen elektrisch umgeschaltet.

In jedem Fall werden die Pumpen 10, 12 von den Steuermitteln 24 so gesteuert, dass jede aktive Pumpe während der Operation mit konstanter und insbesondere mit maximaler Pumpleistung arbeitet. Dies hat den Vorteil, dass bei einem unerwarteten Abfalls des Vakuums in dem zu der Saugringeinheit 18 führenden Teil des Evakuierungswegsystems 16 äußerst rasch reagiert werden kann. Durch sofortiges teilweises der vollständiges Schließen des Bypassventils 20 kann das Vakuum nach einem solchen Abfall unverzüglich wieder angehoben werden. Da die aktive Pumpe bzw. die Pumpen schon auf maximaler Leistung laufen, ist die Reaktionszeit des Gesamtsystems extrem kurz.

## Patentansprüche

1. Einrichtung für die Augenchirurgie, mit einer Vakuumpumpanordnung (10, 12), einem von der Vakuumpumpanordnung ausgehenden Evakuierungswegsystem (16) zum Anschluss an eine auf das Auge aufzusetzende Komponente (18) und Drucksteuermitteln (24) zur Steuerung des Drucks in dem Evakuierungswegsystem, **gekennzeichnet durch** eine stromabwärts der Vakuumpumpanordnung (10, 12) in das Evakuierungswegsystem (16) eingefügte steuerbare Fremdlufteinleitungsstelle (22), wobei die Drucksteuermittel (24) mit der Fremdlufteinleitungsstelle gekoppelt sind und dazu eingerichtet sind, zur Steuerung des Drucks in dem Evakuierungswegsystem (16) den Fremdlufteinleitungsquerschnitt der Fremdlufteinleitungsstelle (22) zu beeinflussen.

2. Einrichtung nach Anspruch 1,
**dadurch gekennzeichnet, dass** der Fremdlufteinleitungsquerschnitt der Fremdlufteinleitungsstelle (22) stufenlos variierbar ist.

3. Einrichtung nach Anspruch 1 oder 2,
**dadurch gekennzeichnet, dass** die Vakuumpumpanordnung zwei parallel geschaltete Vakuumpumpen (10, 12) umfasst.

4. Einrichtung nach Anspruch 3,
**gekennzeichnet durch** einen stromabwärts der Vakuumpumpanordnung (10, 12), jedoch stromaufwärts der Fremdlufteinleitungsstelle (22) in das Evakuierungswegsystem (16) eingefügten Umschalter (28), mittels welchem wahlweise eine der Vakuumpumpen zuschaltbar ist.

5. Verfahren zum Betreiben einer Einrichtung nach einem der vorhergehenden Ansprüche,
**dadurch gekennzeichnet, dass** mindestens eine Vakuumpumpe (10, 12) der Vakuumpumpanordnung kontinuierlich mit einer vorbestimmten Pumpleistung, insbesondere einer maximalen Pumpleistung, betrieben wird und der Druck in dem Evakuierungswegsystem (16) durch Beeinflussung des Fremdlufteinleitungsquerschnitts der Fremdlufteinleitungsstelle (22) auf einen gewünschten Solldruckwert eingeregelt wird.

## Geänderte Patentansprüche

### Geänderte Patentansprüche gemäss Regel 86(2) EPÜ.

**1.** Einrichtung für die Augenchirurgie, mit einer Vakuumpumpanordnung (10, 12), einem von der Vakuumpumpanordnung ausgehenden Evakuierungswegsystem (16) zum Anschluss an eine auf das Auge aufzusetzende Saugringeinheit (18), Drucksteuermitteln (24) zur Steuerung des Drucks in dem Evakuierungswegsystem und einer stromabwärts der Vakuumpumpanordnung (10, 12) in das Evakuierungswegsystem (16) eingefügten, durch die Drucksteuermittel steuerbaren Fremdlufteinleitungsstelle (22),
**dadurch gekennzeichnet, dass** die Drucksteuermittel dazu eingerichtet sind, den operativen Betriebsdruck in dem Evakuierungswegsystem (16) durch Steuerung des Fremdlufteinleitungsquerschnitts der Fremdlufteinleitungsstelle (22) auf ein Soll-Vakuum einzuregeln.
